(19) **Europäisches Patentamt / European Patent Office / Office européen des brevets**

(11) **EP 2 851 083 B1**

(12) **EUROPEAN PATENT SPECIFICATION**

(45) Date of publication and mention of the grant of the patent:
**20.06.2018 Bulletin 2018/25**

(21) Application number: **13790434.8**

(22) Date of filing: **13.05.2013**

(51) Int Cl.:
*A61L 27/26* (2006.01)   *A61L 27/50* (2006.01)
*A61L 28/00* (2006.01)   *A61L 29/04* (2006.01)
*A61L 31/04* (2006.01)   *A61K 47/38* (2006.01)
*A61L 15/22* (2006.01)   *A61L 17/10* (2006.01)
*A61K 38/00* (2006.01)   *A61K 9/70* (2006.01)
*A61K 38/48* (2006.01)   *A61L 31/14* (2006.01)
*A61K 38/17* (2006.01)

(86) International application number:
**PCT/JP2013/063867**

(87) International publication number:
**WO 2013/172467 (21.11.2013 Gazette 2013/47)**

(54) **RADIATION-STERILIZATION-RESISTANT PROTEIN COMPOSITION**

STRAHLUNGSSTERILISIERUNGSBESTÄNDIGE PROTEINZUSAMMENSETZUNG

COMPOSITION DE PROTÉINE RÉSISTANT À UNE STÉRILISATION PAR RAYONNEMENT

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **14.05.2012   JP 2012110395**
**14.05.2012   JP 2012110764**
**01.03.2013   JP 2013040594**

(43) Date of publication of application:
**25.03.2015   Bulletin 2015/13**

(60) Divisional application:
**18167342.7**

(73) Proprietors:
- **Teijin Limited**
  **Osaka-shi, Osaka 541-0054 (JP)**
- **Teijin Pharma Limited**
  **Tokyo 100-0013 (JP)**
- **The Chemo-Sero-Therapeutic Research Institute**
  **Kumamoto-shi**
  **Kumamoto 860-8568 (JP)**

(72) Inventors:
- **KAGEYAMA, Yukako**
  **Hino-shi**
  **Tokyo 191-0065 (JP)**
- **FUJINAGA, Kentaro**
  **Hino-shi**
  **Tokyo 191-0065 (JP)**
- **YAMAGUCHI, Ayuko**
  **Hino-shi**
  **Tokyo 191-0065 (JP)**
- **AKIYAMA, Yusuke**
  **Hino-shi**
  **Tokyo 191-0065 (JP)**
- **KATOU, Souichirou**
  **Hino-shi**
  **Tokyo 191-0065 (JP)**
- **KIMURA, Yukiko**
  **Hino-shi**
  **Tokyo 191-0065 (JP)**
- **HONDA, Susumu**
  **Hino-shi**
  **Tokyo 191-0065 (JP)**
- **SATAKE, Makoto**
  **Hino-shi**
  **Tokyo 191-0065 (JP)**
- **KANEKO, Hiroaki**
  **Hino-shi**
  **Tokyo 191-0065 (JP)**
- **ISHIWARI, Ayumi**
  **Hino-shi**
  **Tokyo 191-0065 (JP)**
- **HIRASHIMA, Masaki**
  **Kikuchi-shi**
  **Kumamoto 869-1205 (JP)**

**(Cont. next page)**

EP 2 851 083 B1

(74) Representative: **Carpmaels & Ransford LLP**
     **One Southampton Row**
     **London WC1B 5HA (GB)**

(56) References cited:
     **WO-A2-2011/094469   JP-A- H07 252 145**
     **JP-A- 2002 513 645   JP-A- 2002 531 532**

**JP-A- 2005 505 324   JP-A- 2005 505 552**
**JP-A- 2006 508 971   JP-A- 2007 016 049**
**JP-A- 2007 295 936   JP-A- 2008 516 735**
**US-A- 5 730 933**

**Description**

TECHNICAL FIELD

The present invention relates to a protein composition which comprises specific amino acids and/or a cellulose ether derivative and has resistance to radiation sterilization.

BACKGROUND ART

Natural and synthetic proteins are becoming more and more important as drugs. When they are used for medical applications, their products must be sterilized. As means of sterilization, there are known heat sterilization in an autoclave, sterilization with ionizing radiation such as a $\gamma$ ray or electron beam, gas sterilization with an ethylene oxide gas, plasma sterilization with hydrogen peroxide, and separate sterilization using a chemical sterilant comprising a glutaraldehyde formulation or a filter. However, the activities of proteins such as bioactive proteins are reduced by sterilization with heat or radiation. Sterilization with ethylene oxide has possibilities that a by-product may be produced by a chemical reaction and that a highly toxic residual gas may adversely affect the human body. Sterilization with a chemical sterilant has a problem that the resistance to a sterilant of a protein and changes in pH, ion intensity and temperature must be taken into consideration. Then, to manufacture pharmaceuticals and medical products containing or immobilizing a protein, their production processes must be entirely made in sterile conditions and a huge amount of production cost is required.

Although a solution containing a protein is subjected to separate sterilization with a filter, it is difficult to apply this separate sterilization to a composition containing large particles or a solid or semisolid composition.

EP0437095 teaches that a neutralized oxidized cellulose product combined with heparin or a heparin fragment (nORC) can be sterilized by gamma-ray irradiation. However, this document fails to teach the sterilization of ORC or n-ORC to which a protein is bound.

EP0562864 discloses a composite wound care substance containing a collagen sponge matrix, a second bioabsorbable polymer (such as an oxidized regenerated cellulose (ORC) dispersed fiber) and an activator (such as peptide). This document teaches that the activator may be contained in the matrix, the bioabsorbable polymer or both of them and that the composite sponge substance can be sterilized while it is packaged.

US5730933 discloses a method of sterilizing biologically active peptide by gamma-ray or electron-beam irradiation without the loss of the biological activity of the peptide. This method is a technology comprising the steps of forming a mixture of biologically active peptide and a foreign protein such as gelatin, freezing or lyophilizing this mixture, and irradiating it. This document teaches that the existence of the foreign protein stabilizes peptide and prevents the reduction of the activity of peptide.

WO2000/033893 discloses a complex of therapeutic peptide and a polysaccharide selected from the group consisting of oxidized regenerated cellulose, neutralized oxidized regenerated cellulose and mixtures thereof. This document teaches that when peptide is formulated together with an effective amount of the polysaccharide before sterilization with ionizing radiation, the biological activity of the peptide therapeutic agent is not lost and is stabilized if peptide is sterilized with ionizing radiation.

However, these documents do not suggest that the deactivation of a protein at the time of sterilizing with ionizing radiation can be suppressed by making a cellulose ether derivative and specific amino acids coexistent with the protein.

Meanwhile, JP-A 2011-47089 discloses a process for producing an enzyme-containing nanofiber having excellent enzyme activity. In this process, a spinning solution containing an enzyme and a polymer dissolved in a nonaqueous solvent is spun by an electrostatic spinning method to form a zymogen nanofiber which is then imparted with water and dried. However, this document is silent about the sterilization of the enzyme-containing nanofiber.

DISCLOSURE OF THE INVENTION

It is an object of the present invention to provide a protein composition having resistance to radiation sterilization.

The inventors of the present invention conducted intensive studies to solve the above problem and found that, surprisingly, the resistance to radiation sterilization of a protein is improved by making a mixture of glycine, phenylalanine and histidine and/or a cellulose ether derivative coexistent with the protein. The present invention was accomplished based on this finding.

That is, the present invention is a protein composition which comprises a mixture of glycine, phenylalanine and histidine and/or a cellulose ether derivative as an additive.

BRIEF DESCRIPTION OF THE DRAWINGS

Fig. 1 shows the sterilization resisting effect for a protein of a combination of a cellulose ether derivative and specific additives of the present invention (axis of ordinate: gel intensity relative value (before sterilization: 100)); and
Fig. 2 shows the sterilization resisting effect of a combination of a cellulose ether derivative and specific additives of the present invention (axis of ordinate: an increase in the amount of a protein aggregate (%)).

BEST MODE FOR CARRYING OUT THE INVENTION

The present invention is a protein composition which comprises a mixture of glycine, phenylalanine and histidine and/or a cellulose ether derivative as an additive.

The protein used in the present invention is not particularly limited. Examples of the protein include hemostat proteins typified by fibrinogen and thrombin, enzymes typified by asparaginase, catalase, superoxide dismutase and lipase, transport proteins typified by hemoglobin, serum albumin and low density lipoprotein, muscle proteins typified by actin and myosin, defense proteins typified by antibodies and complements, toxin proteins typified by diphtheria toxin, botulinum toxin and snake venom, protein hormones typified by insulin, growth factors and cytokine, storage proteins typified by ovalbumin and ferritin, structural proteins typified by collagen and keratin, and growth factors typified by epidermal growth factor (EGF), insulin-like growth factor (IGF), transforming growth factor (TGF), nerve growth factor (NGF), brain-derived neurotrophic factor (BDNF), vascular endothelial growth factor (VEGF), granulocyte-colony stimulating factor (G-CSF), granulocyte-macrophage-colony stimulating factor (GM-CSF), platelet-derived growth factor (PDGF), erythropoietin (EPO), thrombopoietin (TPO), basic fibroblast growth factor (bFGF or FGF2) and hepatocyte growth factor (HGF). Out of these, hemostat proteins, enzymes, transport proteins, muscle proteins, defense proteins, toxin proteins, protein hormones, storage proteins, structural proteins and growth factors are preferred, and fibrinogen is particularly preferred.

The protein used in the present invention may be of animal origin or manufactured by a genetic recombination technique. If it is of animal origin, it is preferably of human origin. The protein manufactured by the genetic recombination technique may be a variant obtained by replacing the amino acid sequence by another amino acid sequence if the essential bioactivity is the same. Proteins obtained by modifying these proteins and mixtures thereof may also be used.

To the protein used in the present invention, a stabilizer and an additive which are pharmaceutically acceptable (to be referred to as "stabilizer, etc." hereinafter and distinguished from the additive which is made coexistent with the protein to provide resistance to radiation sterilization in the present invention) may be added. Preferred examples of the stabilizer, etc. include arginine, isoleucine, glutamic acid, citric acid, calcium chloride, sodium chloride, protease inhibitors (such as aprotinin), albumin, surfactants, phospholipids, polyethylene glycol, sodium hyaluronate, glycerin, trehalose and sugar alcohols (such as glycerol and mannitol) . At least one selected from arginine, sodium chloride, trehalose, mannitol and citric acid is preferred, and citric acid is particularly preferred.

A mixture of the protein and the stabilizer, etc. used in the present invention contains the protein in an amount of not more than 35 parts by weight, preferably not more than 30 parts by weight based on 100 parts by weight of the mixture.

When the additive used in the present invention is a mixture of glycine, phenylalanine and histidine, the content of glycine is generally 5 to 90 parts by weight, preferably 15 to 60 parts by weight, more preferably 20 to 40 parts by weight, the content of phenylalanine is generally 1 to 80 parts by weight, preferably 2 to 40 parts by weight, more preferably 4 to 20 parts by weight, and the content of histidine is generally 2 to 70 parts by weight, preferably 5 to 40 parts by weight, more preferably 8 to 20 parts by weight based on 100 parts by weight of the total of the additive and the protein.

When the additive in the present invention is a cellulose ether derivative, the protein or a mixture of the protein and the stabilizer, etc. used in the present invention may be supported on the cellulose ether derivative but preferably contained in the cellulose ether derivative (the word "contained" refers to a state that at least part of the protein enters the inside of the cellulose ether derivative) . In this case, the molecules of the protein and the stabilizer, etc. may be dispersed in the cellulose ether derivative but preferably as particles formed by the aggregation of the molecules of the protein and the stabilizer, etc. (may be referred to as "protein particles" including mixed particles with the stabilizer, etc.)

This preferred existence of the protein or the protein particles in the cellulose ether derivative remains unchanged when the additive is only the cellulose ether derivative and also when the additive consists of a mixture of glycine, phenylalanine and histidine and the cellulose ether derivative.

Examples of the cellulose ether derivative used in the present invention include hydroxypropyl cellulose, methyl cellulose, hydroxyethyl cellulose, hydroxypropylmethyl cellulose, sodium carboxymethyl cellulose and mixtures thereof.

One selected from the group consisting of hydroxypropyl cellulose, hydroxyethyl cellulose, hydroxypropylmethyl cellulose and mixtures thereof is preferred, and hydroxypropyl cellulose is most preferred.

Although the molecular weight of the cellulose ether derivative used in the present invention is not particularly limited, when viscosity measurement is carried out at a concentration of 2 % and 20°C, a molecular weight which exhibits a viscosity of 1 to 10,000 mPa·s, preferably 2 to 5,000 mPa·s, more preferably 2 to 4,000 mPa·s is selected.

In the protein composition of the present invention, another polymer or another compound may be used in combination as long as the object of the present invention is not impaired.

The cellulose ether derivative used in the present invention preferably has high purity. Especially, the contents of additives and plasticizer contained in the cellulose ether derivative and residues such as residual catalyst, residual monomers and residual solvent used for molding and post-processing are preferably as low as possible. Especially when the composition is used for medical purposes, it is necessary to reduce these contents to values below safety standards.

The form of the protein composition of the present invention is not limited to a particular form including an indeterminate form, and the composition may be in the form of a film, fiber, sheet, plate-like body, tube-like body, linear body, rod-like body, cushion material, foam or porous body. The molding method for producing a molded product is not particularly limited if it is a method in which the activity of the protein is not reduced. For example, suitable molding techniques such as extrusion molding, injection molding, calender molding, compression molding, blow molding, vacuum forming, powder molding, cast molding and casting may be employed. The protein composition of the present invention is suitable for the production of films and fibers. The fiber form as used herein refers to a 3-D molded body formed by the lamination, weaving, knitting or another technique of one or a plurality of fibers. The fiber form is, for example, a nonwoven fabric. Further, a tube and a mesh obtained by processing the nonwoven fabric are included in the fiber form.

For the production of these, any one of techniques which have been employed for the production of films or plastic fibers may be employed. For example, extrusion molding techniques such as casting, electrospinning, inflation extrusion molding and T die extrusion molding, and calendering technique may be used. The above molding may be solution molding or melt molding, out of which solution molding is preferred in order to facilitate the dispersion of the protein or the protein particles so as to prevent the functional deterioration of the protein.

The process for producing the protein composition having a film form out of the present invention will be explained, taking the casting technique as an example. Protein particles having an average particle diameter (generally 0.1 to 200 $\mu$m, preferably 1 to 100 $\mu$m) suitable for dispersion in a solvent are prepared by pounding lyophilized protein powders in a mortar. After the protein particles are dispersed in one or more suitable solvents (such as 2-propanol and ethanol) which can dissolve the cellulose ether derivative, can form a suspension with the protein particles and evaporate in the film forming step to form a film, the cellulose ether derivative and further optionally a plasticizer such as MACROGOL are dissolved in the resulting dispersion so as to prepare a dope solution containing the protein particles dispersed in the cellulose ether derivative solution. A film is formed by the casting technique using the obtained dope solution.

The protein composition having a film form out of the present invention comprises the protein or the protein particles in an amount of generally not less than 100 wt%, preferably not less than 500 wt%, more preferably 800 to 950 wt% based on the cellulose ether derivative though this depends on the type of the protein and the type of the cellulose ether derivative. When the content of the protein or the protein particles falls below the above range, the function of the protein may not be obtained fully and when the content exceeds the above range, film moldability may become unsatisfactory.

The average thickness of a film of the protein composition having a film form out of the present invention which differs according to the intended use is preferably 10 to 1, 000 $\mu$m.

The average fiber diameter of the protein composition having a fiber form out of the present invention is, for example, 0.01 to 50 $\mu$m and may be suitably determined by a person skilled in the art according to the intended use. The protein composition may be in the form of a long fiber. The long fiber is a fiber formed without adding the step of cutting a fiber in the course of transition from spinning to the processing of a fiber molded body. It can be formed by electrospinning, span bonding and melt blowing methods. Out of these, the electrospinning method is preferred as the long fiber can be molded without adding heat and the functional deterioration of the protein can be suppressed.

The electrospinning method is a method in which a fiber molded body is obtained on an electrode by applying a high voltage to a solution containing a polymer. This process comprises the steps of preparing a spinning solution containing a polymer, applying a high voltage to the solution, jetting the solution, forming a fiber molded body by evaporating the solvent from the jetted solution, eliminating the charge of the formed fiber molded body as an optional step, and accumulating the fiber molded body by the charge loss.

A description is subsequently given of the process for producing the protein composition having a fiber form or a nonwoven fabric form out of the present invention, taking the electrospinning method as an example.

The step of preparing a spinning solution in the electrospinning method will be explained. A suspension of a cellulose ether derivative solution and protein particles is preferably used as the spinning solution in the present invention.

The concentration of the cellulose ether derivative in the suspension is preferably 1 to 30 wt%. When the concentration of the cellulose ether derivative is lower than 1 wt%, it is difficult to form a fiber molded body disadvantageously. When the concentration is higher than 30 wt%, the fiber diameter of the obtained fiber molded body becomes large and the viscosity of the suspension becomes high disadvantageously. The concentration of the cellulose ether derivative in the suspension is more preferably 1.5 to 20 wt%.

The solvent for the cellulose ether derivative is not particularly limited if it can dissolve the cellulose ether derivative, forms a suspension with the protein particles and evaporates in the spinning step so that a fiber can be formed. Only one solvent or a combination of two or more solvents may be used. Examples of the solvent include chloroform, 2-propanol, toluene, benzene, benzyl alcohol, dichloromethane, carbon tetrachloride, cyclohexane, cyclohexanone, trichloroethane, methyl ethyl ketone, ethyl acetate, acetone, ethanol, methanol, tetrahydrofuran, 1,4-dioxane, 1-propanol, phenol, pyridine, acetic acid, formic acid, hexafluoro-2-propanol, hexafluoroacetone, N,N-dimethylformamide, N,N-dimethylacetamide, acetonitrile, N-methyl-2-pyrrolidinone, N-methylmorpholine-N-oxide, 1,3-dioxolan, water and mixtures thereof. Out of these, 2-propanol or ethanol is preferably used from the viewpoints of handling ease and physical properties.

Although the method of preparing a suspension by mixing together the cellulose ether derivative solution and the protein particles is not particularly limited, ultraviolet waves or stirring means may be used. As the stirring means, high-speed stirring means such as a homogenizer or stirring means such as an attriter or ball mill may be used. Out of these, dispersion with ultrasonic waves is preferred.

Also, the spinning solution may be prepared by adding the cellulose ether derivative after a suspension is formed from a solvent and the protein particles.

Before the preparation of the suspension, protein particles may be microfabricated. For microfabrication, there are dry milling and wet milling both of which may be employed and also may be combined in the present invention.

Dry milling is carried out by milling with a ball mill, planetary mill or oscillating mill, by pounding in a mortar with a pestle, or by grinding with a medium stirring type pulverizer, jet mill or stone mill.

Meanwhile, wet milling is carried out by stirring with a stirrer or kneader having high shear force while the protein particles are dispersed in a suitable dispersion medium, or by using a ball mill or bead mill while the protein particles are dispersed in a medium.

Further, protein particles produced by a spay drier may also be used.

In the protein composition having a fiber form or a nonwoven fabric form out of the present invention, the sizes of the protein particles are not particularly limited but preferably 0.01 to 100 $\mu$m. It is technically difficult to manufacture protein particles having a particle size smaller than 0.01 $\mu$m, and when the particle size is larger than 100 $\mu$m, dispersibility degrades and the fiber molded body becomes brittle disadvantageously.

The sterilization method used for the protein composition of the present invention is radiation sterilization. Examples of the radiation include alpha rays, beta rays, gamma rays, neutron rays, electron beams and X-rays. Out of these, gamma rays and electron beams are preferred, and electron beams are most preferred. Although the sterilization method is not particularly limited, the dose of the radiation is 10 to 80 kGy, preferably 20 to 30 kGy. Although the temperature condition is not particularly limited, it is -80 to 40°C, preferably -80 to 30°C.

The radiation such as alpha rays, positron, gamma rays, neutron rays, electron beams or X-rays strips an electron off from molecules or atoms constituting a substance when it is applied to the substance. A molecular bond is broken upon this, and a highly reactive radical is produced and chemically reacts with a surrounding substance secondarily.

It is well known that a protein tends to lose its function (activity) upon exposure to radiation. This is considered to be due to the destruction of "a high-order structure" which is a source of developing a function by the breakage of a molecular bond by exposure. However, the functional deterioration of the protein composition of the present invention is suppressed even when it is exposed to radiation. This means that the high-order structure of the protein is retained in the composition of the present invention, which is a common effect regardless of the type of the protein. It is not considered from the thickness of the cellulose ether derivative through which the radiation is transmitted that this effect is due to screening, and the control mechanism is not known. The mechanism of a phenomenon that the effect of the cellulose ether derivative is remarkably improved by the addition of specific amino acids is not known as well.

The protein composition of the present invention may further comprise an electron/ion scavenger, energy transfer agent, radical scavenger, antioxidant and plasticizer. Examples of the electron/ion scavenger include N,N'-tetramethyl phenylenediamine, diphenylenediamine, pyrene and quinone. Examples of the energy transfer agent include acenaphthene. Examples of the radical scavenger include mercaptans, octahydrophenanthrene, monoalkyl diphenyl ethers, tocopherol, citric acid, butylated hydroxyanisole, butylated hydroxytoluene, t-butyl hydroquinone, propyl gallate and ascorbic acid derivatives. Examples of the antioxidant include BHT, phosphite triesters, phenolic antiaging agents and organic thio acid salts. Additives that are generally accepted as safe for use in foods and pharmaceuticals are preferred. The amount of the additive which is not particularly limited is, for example, 0.01 to 10 wt% based on the cellulose ether derivative in the protein composition.

The cellulose ether derivative containing the protein in the sterilization step preferably contains no water. The water content of the cellulose ether derivative is preferably not more than 10 wt%, more preferably not more than 4 wt%, much more preferably substantially 0 wt%.

The protein composition of the present invention may be wrapped in a packaging material to be sterilized with radiation. As the packaging material, a material having high gas barrier properties such as aluminum is preferably used. The protein composition may be hermetically sealed and packaged together with a deoxidant or desiccant or while an inert gas is filled into the package after degasification, or both methods may be combined together. As the deoxidant and the desiccant, ones which do no harm to the human body and are not deactivated upon exposure to radiation are preferred.

The protein composition of the present invention may be used as a medical material which requires the function and sterility of a protein.

The present invention includes a sterile protein composition obtained by sterilizing the protein composition of the present invention with radiation.

EXAMPLES

The following examples are provided for the purpose of further illustrating the present invention but are in no way to be taken as limiting.

Measurement methods for Examples 1 to 4 and Comparative Examples 1 and 2

1. Average fiber diameter:

The diameters of fibers at 20 locations selected at random from a photo of the surface of the obtained fiber molded body taken by a scanning electron microscope (VE8800 of Keyence Corporation) at 3,000-fold magnification to obtain the average value of all the fiber diameters as average fiber diameter. N = 20.

2. Average thickness:

The film thicknesses of 15 fiber molded bodies cut to a size of 50 mm x 100 mm were measured with a measurement force of 0.01 N by means of a high-resolution digimatic measuring unit (LITEMATIC VL-50 of Mitutoyo Corporation) to calculate the average value. This measurement was carried out with minimum measurement force that could be used by the measuring unit.

3. ELISA measurement

(1) Fibrinogen

10 $\mu$g/mL of an antihuman fibrinogen antibody (DAKO A0080) was immobilized to an ELISA plate (NUNC 468667). After it was washed with PBS containing 0.05 % of Tween 20, Block Ace (UK-B80 of DS Pharma Biomedical Co., Ltd.) was added to each well to carry out masking. After washing with PBS containing 0.05 % of Tween 20, a test body was added. Human fibrinogen (No. FIB3 of Enzyme Research Laboratories) was used as a standard to form a calibration curve. After washing with PBS containing 0.05 % of Tween 20, an HRP-labelled antihuman fibrinogen antibody (CPL5523) was added. After a reaction, the reaction product was washed with PBS containing 0.05 % of Tween 20, a TMB reagent (KPL 50-76-02 50-65-02) was added, and the resulting mixture was left for 6 minutes to develop color. 1 M $H_3PO_4$ was added to stop color development so as to measure OD450-650 nm with a microplate reader.

(2) Thrombin

5 $\mu$g/mL of an antihuman thrombin antibody (No. SAHT-AP of Affinity Biologicals Inc.) was immobilized to an ELISA plate (NUNC 468667) . After it was washed with PBS containing 0.05 % of Tween 20, Block Ace (UK-B80 of DS Pharma Biomedical Co., Ltd.) was added to each well to carry out masking. After washing with PBS containing 0.05 % of Tween 20, a test body was added. Human thrombin (HCT-0020 of Haematologic Technologies, Inc.) was used as a standard to form a calibration curve. After washing with PBS containing 0.05 % of Tween 20, 0.1 $\mu$g/mL of an HRP-labelled antihuman thrombin antibody (No. SAHT-HRP of Affinity Biologicals Inc.) was added. After a reaction, the reaction product was washed with PBS containing 0.05 % of Tween 20, a TMB reagent (DaKo S1599) was added, and the resulting mixture was left for 10 minutes to develop color. 0. 5M $H_2SO_4$ was added to stop color development so as to measure OD450-650 nm with a microplate reader.

4. Measurement of thrombin activity

20 $\mu$L of a sample and 80 $\mu$L of a dilution solution for activity measurement (0.01 % F-68, 50 mmol/L NaCl, 50 mmol/L Tris-HCl, pH 8.4) were added to the polystyrene tube of BD to be incubated at 37°C for 3 minutes. Recombinant thrombin (JPU Thrombin Standard 400 U/mL or WHO/US Thrombin Standard 110 IU/mL: prepared by their own companies) diluted with the above buffer to 4, 2, 1, 0.5 and 0.25 U/mL in the case of JPU and to 6, 3, 1.5, 0.75 and 0.375 IU/mL in the case of IU was used as a standard. 100 μL of the S-2238 test team chromogenic substrate (1 mM: Daiichi Pure Chemicals Co., Ltd.) was added to and mixed with the obtained reaction solution under agitation to carry out a reaction at 37°C for 7 minutes, and then 800 μL of a 0.1 M citric acid solution was added to terminate the reaction. 200 μL of the reaction solution was transferred to 96 well plates to measure OD405/650.

Example 1

After lyophilized fibrinogen powders (Bolheal, (registered trademark, the same shall apply hereinafter), tissue adhesive: Vial 1) were dispersed in 2-propanol, hydroxypropyl cellulose (6-10 mPa·s, manufactured by Wako Pure Chemical Industries, Ltd.) was dissolved in the resulting dispersion to a concentration of 16 wt% so as to prepare a spinning solution having a fibrinogen-containing particle/hydroxypropyl cellulose ratio of 20 (9.2 as fibrinogen)/100 (w/w). Spinning was carried out by an electrospinning method at a temperature of 22°C and a humidity of not more than 26% to obtain a sheet-like fiber molded body. The inner diameter of a jet nozzle was 0.8 mm, the voltage was 11 kV, the flow rate of the spinning solution was 1.2 mL/h, and the distance from the jet nozzle to a flat plate was 15 cm. The obtained fiber molded body had an average fiber diameter of 0.86 μm and an average thickness of 137 μm. The obtained sheet was sterilized with a 20 kGy electron beam. The sterilized sheet was cut to a size of 0.5 cm x 0.5 cm, and the protein was extracted with 62.5 μL of physiological saline to carry out ELISA measurement. As a result, the amount of the immobilized protein was 0.15 mg/cm$^2$. Meanwhile, when ELISA measurement was made on an unsterilized sheet likewise, the amount of the immobilized protein was 0.16 mg/cm$^2$. Therefore, the recovery rate of the protein of the sterilized sheet was 94 % of that of the unsterilized sheet.

Example 2

After lyophilized fibrinogen powders (Bolheal tissue adhesive: Vial 1) were dispersed in 2-propanol, hydroxypropyl cellulose (6-10 mPa·s, manufactured by Wako Pure Chemical Industries, Ltd.) was dissolved in the resulting dispersion to a concentration of 16 wt% so as to prepare a spinning solution having a lyophilized fibrinogen powder/hydroxypropyl cellulose ratio of 40 (18 as fibrinogen)/100 (w/w). Spinning was carried out by the electrospinning method at a temperature of 22°C and a humidity of not more than 26% to obtain a sheet-like fiber molded body. The inner diameter of the jet nozzle was 0.8 mm, the voltage was 12.5 kV, the flow rate of the spinning solution was 1.2 mL/h, and the distance from the jet nozzle to the flat plate was 15 cm. The obtained fiber molded body had an average fiber diameter of 0.43 μm and an average thickness of 152 μm. The obtained sheet was sterilized with a 20 kGy electron beam. The sterilized sheet was cut to a size of 0.5 cm x 0.5 cm, and the protein was extracted with 62.5 μL of physiological saline to carry out ELISA measurement. As a result, the amount of the immobilized protein was 0.27 mg/cm$^2$. Meanwhile, when ELISA measurement was made on an unsterilized sheet likewise, the amount of the immobilized protein was 0.30 mg/cm$^2$. Therefore, the recovery rate of the protein of the sterilized sheet was 90 % of that of the unsterilized sheet.

Example 3

After lyophilized fibrinogen powders (Bolheal tissue adhesive: Vial 1) were dispersed in 2-propanol, hydroxypropyl cellulose (6-10 mPa·s, manufactured by Wako Pure Chemical Industries, Ltd.) was dissolved in the resulting dispersion to a concentration of 16 wt% so as to prepare a spinning solution having a lyophilized fibrinogen powder/hydroxypropyl cellulose ratio of 100 (46 as fibrinogen)/100 (w/w). Spinning was carried out by the electrospinning method at a temperature of 22°C and a humidity of not more than 26% to obtain a sheet-like fiber molded body. The inner diameter of the jet nozzle was 0.8 mm, the voltage was 12.5 kV, the flow rate of the spinning solution was 1.2 mL/h, and the distance from the jet nozzle to the flat plate was 15 cm. The obtained fiber molded body had an average fiber diameter of 0.35 μm and an average thickness of 191 μm. The obtained sheet was sterilized with a 20 kGy electron beam. The sterilized sheet was cut to a size of 0.5 cm x 0.5 cm, and the protein was extracted with 62.5 μL of physiological saline to carry out ELISA measurement. As a result, the amount of the immobilized protein was 0.78 mg/cm$^2$. Meanwhile, when ELISA measurement was made on an unsterilized sheet likewise, the amount of the immobilized protein was 0.76 mg/cm$^2$. Therefore, the recovery rate of the protein of the sterilized sheet was 102 % of that of the unsterilized sheet.

Comparative Example 1

Lyophilized fibrinogen powders (Bolheal tissue adhesive: Vial 1) were sterilized with a 20 kGy electron beam. The protein was extracted with 1 mL of physiological saline to carry out ELISA measurement. As a result, the ELISA measurement value was 31 μg/mL. Meanwhile, when ELISA measurement was made on unsterilized lyophilized fibrinogen powders (Bolheal) likewise, the ELISA measurement value was 90 μg/mL. Therefore, the recovery rate of the protein of the sterilized sheet was 34 % of that of the unsterilized sheet.

Example 4

After thrombin-containing particles (prepared by lyophilizing an aqueous solution containing 1 mg/mL of recombinant thrombin, sodium chloride, sodium citrate, calcium chloride and mannitol and having a pH of 7) were dispersed in 2-propanol, hydroxypropyl cellulose (2.0-2.9 mPa·s, manufactured by Nippon Soda Co., Ltd.) was dissolved in the resulting dispersion to a concentration of 13 wt% so as to prepare a dope solution having a thrombin-containing particle/hydroxypropyl cellulose ratio of 100/100 (w/w). Spinning was carried out by the electrospinning method to obtain a sheet-like fiber molded body. The obtained fiber molded body had a thickness of 204 $\mu$m, a weight of 2.08 mg/cm$^2$ and a bulk density of 101 mg/cm$^3$. The obtained sheet was cut to a diameter of 1 cm, and the protein was extracted with 200 $\mu$L of physiological saline to measure its activity. As a result, the activity measurement value was 110.3 U/cm$^2$. The obtained sheet was sterilized by exposure to a 30 kGy electron beam to measure the activity of thrombin. When the activity of thrombin before sterilization was 100 %, the retention rate of the activity of thrombin right after exposure to an electron beam was 68.4 %.

Comparative Example 2

After a 30 kGy electron beam was applied to thrombin-containing particles (prepared by lyophilizing an aqueous solution containing 1 mg/mL of recombinant thrombin, sodium chloride, sodium citrate, calcium chloride and mannitol and having a pH of 7) to sterilize them, the activity of thrombin was measured. The activity of thrombin before exposure was 404.73 U/vial. When the activity of thrombin before sterilization was 100 %, the retention rate of the activity of thrombin right after exposure to an electron beam was 51.8 %.

Measurement methods for Examples 5 and 6 and Comparative Examples 3 and 4

1. Average thickness:

The film thicknesses of 9 fiber molded bodies obtained by cutting the composition to a suitable size were measured with a measurement force of 0.01 N by means of a high-resolution digimatic measuring unit (LITEMATIC VL-50 of Mitutoyo Corporation) to calculate the average value. This measurement was carried out with minimum measurement force that could be used by the measuring unit.

2. Measurement of enzyme activity

A continuous fluorometric lipase test kit (manufactured by PROGEN BIOTECHNIK GMBH) was used to measure the activity of lipase. The retention rate of activity was calculated from the following equation. The amount of the active enzyme was calculated in terms of concentration from the value of activity.

```
Retention rate of activity (%) = {amount of active enzyme
after sterilization (mg/cm²) /amount of active enzyme before
sterilization (mg/cm²)} x 100
```

Fluorescent measurement using Tokyogreen (registered trademark, the same shall apply hereinafter)-$\beta$Glu (of Sekisui Medical Co., Ltd.) was employed to measure the activity of $\beta$-glucosidase. The recovery rate of activity was calculated from the following equation. The theoretical weight of the immobilized enzyme was calculated from wt% of the charged enzyme powder and the weight of the composition.

```
Recovery rate of activity (%) = {amount of active enzyme
(mg)/theoretical weight of immobilized enzyme (mg)} x 100
```

The retention rate of activity was calculated from the following equation.

```
Retention rate of activity (%) = {recovery rate of activity
after sterilization (%)/recovery rate of activity before
sterilization (%))} x 100
```

Example 5

After lipase powders (derived from pig pancreas, manufactured by Wako Pure Chemical Industries, Ltd., the same shall apply hereinafter) were dispersed in 2-propanol, hydroxypropyl cellulose (6-10 mPa·s, manufactured by Wako Pure Chemical Industries, Ltd.) was dissolved in the resulting dispersion to a concentration of 13 wt% so as to prepare a spinning solution having a lipase powder/hydroxypropyl cellulose ratio of 50/100 (w/w). Spinning was carried out by the electrospinning method at a temperature of 27°C and a humidity of not more than 27 % to obtain a sheet-like fiber molded body. The inner diameter of the jet nozzle was 0.8 mm, the voltage was 18 kV, the flow rate of the spinning solution was 1.2 mL/h, and the distance from the jet nozzle to the flat plate was 16.5 cm. The obtained fiber molded body (10cm x 14 cm) had an average thickness of 168 $\mu$m. The obtained fiber molded body was sterilized with a 20 kGy electron beam. After the sterilized fiber molded body was cut to a size of 1 cm x 1 cm, lipase was extracted with 1 mL of a lipase buffer contained in a kit to measure its activity. As a result, the amount of the active enzyme was 0.46 mg/cm$^2$. Meanwhile, when activity measurement was made on an unsterilized sheet likewise, the amount of the active enzyme was 0.40 mg/cm$^2$. It is understood from above that the retention rate of the activity of the sterilized fiber molded body was 115 % of that of the unsterilized fiber molded body and that lipase was not deactivated by sterilization with an electron beam.

Example 6

After lipase powders were dispersed in 2-propanol, hydroxypropyl cellulose (6-10 mPa·s, manufactured by Wako Pure Chemical Industries, Ltd.) was dissolved in the resulting dispersion to a concentration of 13 wt% so as to prepare a cast solution having a lipase powder/hydroxypropyl cellulose ratio of 50/100 (w/w). Casting was carried out by using a doctor blade (YBA-3 of YOSHIMITSU) at a coating width of 15 mil to obtain a sheet. The obtained sheet (4 cm x 6 cm) had an average thickness of 180 $\mu$m. The obtained sheet was sterilized with a 20 kGy electron beam. After the sterilized sheet was cut to a size of 1 cm x 1 cm, lipase was extracted with 1 mL of a lipase buffer contained in a kit to measure its activity. As a result, the amount of the active enzyme was 0.69 mg/cm$^2$. Meanwhile, when activity measurement was made on an unsterilized sheet likewise, the amount of the active enzyme was 0.64 mg/cm$^2$. It is understood from above that the retention rate of the activity of the sterilized sheet was 108 % of that of the unsterilized sheet and that lipase was not deactivated by sterilization with an electron beam.

Example 7

After $\beta$-glucosidase powders (derived from almond, manufactured by Oriental Yeast Co., Ltd, the same shall apply hereinafter) were dispersed in 2-propanol, hydroxypropyl cellulose (6-10 mPa·s, manufactured by Wako Pure Chemical Industries, Ltd.) was dissolved in the resulting dispersion to a concentration of 13 wt% so as to prepare a spinning solution having a $\beta$-glucosidase powder/hydroxypropyl cellulose ratio of 38/62 (w/w). Spinning was carried out by the electrospinning method at a temperature of 27°C and a humidity of not more than 27 % to obtain a sheet-like fiber molded body. The inner diameter of the jet nozzle was 0.9 mm, the voltage was 18 kV, the flow rate of the spinning solution was 1.2 mL/h, and the distance from the jet nozzle to the flat plate was 16.5 cm. The obtained fiber molded body (10 cm x 10 cm) had an average thickness of 207 $\mu$m. After the obtained fiber molded body was cut to a size of 2 cm x 2 cm, it was sterilized with a 20 kGy electron beam. $\beta$-glucosidase was extracted from the obtained sterilized sheet with 1 mL of physiological saline to measure its activity with Tokyogreen-$\beta$Glu. As a result, the recovery rate of activity was 42 %. Meanwhile, when activity measurement was made on an unsterilized sheet likewise, the recovery rate of activity was 46 %. It is understood from above that the retention rate of the activity of the sterilized fiber molded body was 91 % of that of the unsterilized fiber molded body and that the deactivation of the enzyme can be suppressed by containing it in the cellulose ether derivative.

Comparative Example 3

Lipase powders were sterilized with a 20 kGy electron beam. 1 mL of a lipase buffer was added to 1 mg of the powders to measure its activity. As a result, the activity value was 0.25 pmol/mL·min. Meanwhile, when activity measurement was made on unsterilized lipase powders likewise, the activity value was 0.34 pmol/mL·min. Therefore, the retention rate of the activity of the sterilized powders was 74 % of that of the unsterilized powders.

Comparative Example 4

β-glucosidase powders were sterilized with a 20 kGy electron beam. 2 mg of the powders was dissolved in 1 mL of physiological saline to measure its activity with Tokyogreen-βGlu. As a result, the retention rate of activity was 81 %.

Measurement method for Examples 8 to 10

When an electron beam is applied to lyophilized fibrinogen powders (fibrinogen-containing particles), the changes of fibrinogen (increase in the amount of its aggregate, reduction in gel strength) occur. To investigate the effect of suppressing the changes of fibrinogen by exposure to an electron beam (sterilization resisting effect), a bulk solution of fibrinogen was prepared and 1 mL of the bulk solution was charged into a 5 mL glass vial to be lyophilized. A 30 kGy electron beam was applied to part of the vial in which lyophilization was completed to compare each lyophilized product before and after sterilization.

Comparison evaluation was carried out by measuring gel strength by means of the EZTest small-sized bench-top tester (of Shimadzu Corporation) and the content of the aggregate by means of BioSep-SEC-s4000 (of Phenomenex) (analyzing conditions: fractionating with a 50 mM phosphoric acid buffer solution (pH of 7.0) and 0.5 M arginine hydrochloride salt as mobile phases at a flow rate of 1.0 ml/min, detecting a target substance with a wavelength of 280 nm; and determining the quantity of the aggregate from a peak detected earlier than a monomer peak).

As for the procedure of preparing a sample for analysis (analytical sample), an unsterilized lyophilized product vial and a sterilized lyophilized product vial were each dissolved in 1 mL of distilled water. The resulting solutions were centrifuged by a centrifugal tube at 15,000 rpm for 5 minutes and let pass through a 0.45 μm filter to be used as analytical samples.

Example 8

The sterilization resisting effect for a protein of a combination of a cellulose ether derivative and specific additives was investigated by the following method. (method) The function of fibrinogen was evaluated by measuring the gel strength of each of fibrinogen bulk solutions of compositions comprising "a cellulose ether derivative + specific additives" (compositions (1) shown in Nos. 1 to 6 in Table 1 below) and fibrinogen bulk solutions of compositions (2) prepared by eliminating the cellulose ether derivative from the compositions (1), and the gel strengths before and after sterilization of these solutions were compared with each other to investigate the sterilization resisting effect. The results are shown in Table 2.

The compositions (1) (lyophilized powders and hydroxypropyl cellulose were suspended in 2-propanol to form a sheet) and the compositions (2) (lyophilized powders) were dissolved in water to an Fbg concentration of 1 % and diluted with a buffer solution containing 10 mM arginine and 270 mM sodium chloride and having a pH of 8.5 to a concentration of 2 mg/mL.

After 10 μL of fibrogammin (240 units/mL) and 110 μL of a thrombin solution (containing 0.2 mg/mL of 100 mM calcium chloride) were added to a 2 mL polypropylene tube and the resulting solution was pipetted, 900 μL of a 2 mg/mL fibrinogen solution was added in such a manner that air bubbles were not contained and left to stand at 37°C for 1 hour to measure the gel strength by means of the EZTest small-size bench -top tester (of Shimadzu Corporation).

Table 1 compositions (1): compositions comprising cellulose ether derivative + specific additives

| Composition | composition of bulk solution |
| --- | --- |
| No. 1 | 1 % of Fbg, 10 mM arginine, 110 mM sodium chloride, 1.0 % of glycine, 0.1 % of mannitol, 0.4 % of hydroxypropyl cellulose |
| No. 2 | 1 % of Fbg, 10 mM arginine, 110 mM sodium chloride, 1.0 % of glycine, 0.2 % of mannitol, 0.4 % of hydroxypropyl cellulose |
| No. 3 | 1 % of Fbg, 10 mM arginine, 110 mM sodium chloride, 1.0 % of glycine, 0.25 % of phenylalanine, 0.2 % of trehalose, 0.4 % of histidine, 0.1 % of trisodium citrate, 0.4 % of hydroxypropyl cellulose |
| No. 4 | 1 % of Fbg, 10 mM arginine, 110 mM sodium chloride, 1.0 % of glycine, 0.1 % of mannitol, 0.25 % of phenylalanine, 0.2 % of trehalose, 0.4 % of histidine, 0.1 % of trisodium citrate, 0.4 % of hydroxypropyl cellulose |

(continued)

| Composition | composition of bulk solution |
|---|---|
| No. 5 | 1 % of Fbg, 10 mM arginine, 110 mM sodium chloride, 1.0 % of glycine, 0.1 % of mannitol, 0.25 % of phenylalanine, 0.4 % of histidine, 0.1 % of trisodium citrate, 0.4 % of hydroxypropyl cellulose |
| No. 6 | 1 % of Fbg, 10 mM arginine, 110 mM sodium chloride, 1.0 % of glycine, 0.2 % of mannitol, 0.25 % of phenylalanine, 0.4 % of histidine, 0.1 % of trisodium citrate, 0.4 % of hydroxypropyl cellulose |

Compositions (2): compositions comprising specific additives

These were prepared by eliminating the cellulose ether derivative (hydroxypropyl cellulose: HPC) from the compositions (1).

(results)

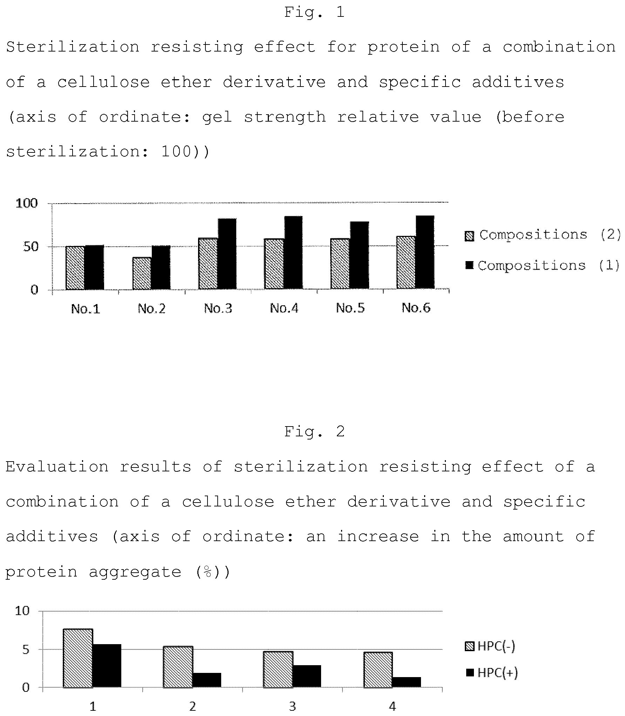

The values of gel strength after sterilization are shown in Table 2 and Fig. 1 when the values before sterilization are 100.

Table 2 sterilization resisting effect for protein of a combination of cellulose ether derivative and specific additives

| composition | Compositions (1) (cellulose ether derivative + specific additives) | compositions (2) (specific additives) |
|---|---|---|
| No. 1 | 51. 5 | 49.8 |
| No. 2 | 51.1 | 36.5 |
| No. 3 | 81. 5 | 58.1 |
| No. 4 | 84.0 | 57.9 |
| No. 5 | 77.6 | 57.7 |
| No. 6 | 84.4 | 59.6 |

The sterilization resistance improving effect due to the existence of the cellulose ether derivative was not observed in the composition No. 1 whereas the above effect due to the existence of the cellulose ether derivative was observed in the compositions Nos. 2 to 6. This effect was marked especially in the composition Nos. 3 to 6.

Example 9

The sterilization resisting effect of glycine was investigated with the compositions (two) shown in Table 3 below in the same manner as in Example 8. The results are shown in Table 4.

Table 3 compositions for evaluating the sterilization resisting effect of glycine

| Composition | fibrinogen | Arginine (pH 8.5) | sodium chloride | mannitol | glycine |
|---|---|---|---|---|---|
| G (-) | 1.0% | 10 mM | 110 mM | 0.2% | 0% |
| G (+) | 1.0% | 10 mM | 110 mM | 0.2% | 1.0% |

Table 4 results of evaluating the sterilization resisting effect of glycine

| Composition | s4000 (aggregate) | | |
|---|---|---|---|
| | Before sterilization (%) | after sterilization (%) | increase in amount (%) |
| G (-) | 14.0 | 28.0 | 14.0 |

(continued)

| Composition | s4000 (aggregate) | | |
|---|---|---|---|
| | Before sterilization (%) | after sterilization (%) | increase in amount (%) |
| G (+) | 14.0 | 21. 6 | **7.6** |

An increase in the content of the fibrinogen aggregate was suppressed by the addition of glycine.

Example 10

The sterilization resisting effect of a combination of a cellulose ether derivative and specific additives was investigated by the same method as in Example 8.

Hydroxypropyl cellulose (HPC) as the cellulose ether derivative and eight different fibrinogen bulk solutions shown in Table 5 below were used. 1.0 % of fibrinogen, 110 mM sodium chloride, 1.0 % of glycine and 0.2 % of mannitol were used in the following eight compositions. The results are shown in Table 6 and Fig. 2.

Table 5 sterilization resisting effect of a combination of cellulose ether derivative and specific additives

| Composition | Arginine (pH8.5) | tris (pH8.5) | histidine | phenylalanine | sodium citrate | HPC |
|---|---|---|---|---|---|---|
| 1HPC (-) | 10mM | 0mM | 0% | 0% | 0% | 0% |
| 1HPC (+) | 10mM | 0mM | 0% | 0% | 0% | 0.419% |
| 2HPC (-) | 10mM | 0mM | 0.4% | 0.25% | 0% | 0% |
| 2HPC (+) | 10mM | 0mM | 0.4% | 0.25% | 0% | 0.419% |
| 3HPC (-) | 10mM | 0mM | 0.4% | 0.25% | 0.1% | 0% |
| 3HPC (+) | 10mM | 0mM | 0.4% | 0.25% | 0.1% | 0.419% |
| 4HPC (-) | 0.4mM | 10mM | 0.4% | 0.25% | 0.1% | 0% |
| 4HPC (+) | 0.4mM | 10mM | 0.4% | 0.25% | 0.1% | 0.419% |

Table 6 evaluation results of sterilization resisting effect of a combination of cellulose ether derivative and specific additives

| Composition | s4000 (aggregate) | | |
|---|---|---|---|
| | Before sterilization (%) | after sterilization (%) | increase in amount (%) |
| 1HPC (-) | 14.0 | 21.6 | 7.6 |
| 1HPC (+) | 12.8 | 18.4 | **5.7** |
| 2HPC (-) | 13.8 | 19.2 | 5.4 |
| 2HPC (+) | 13.9 | 15.8 | **1.9** |
| 3HPC (-) | 15.1 | 19.8 | 4.7 |
| 3HPC (+) | 14.8 | 17.8 | **2.9** |
| 4HPC (-) | 14.9 | 19.5 | 4.6 |
| 4HPC (+) | 14.2 | 15.5 | **1.3** |

An increase in the content of the protein aggregate was suppressed by the addition of the cellulose ether derivative. The effect of suppressing an increase in the above content due to the existence of the cellulose ether derivative was marked in compositions 2 to 4 comprising phenylalanine and histidine. It is understood from this that the reason that the effect of improving sterilization resistance due to the existence of the cellulose ether derivative is not observed in composition No.1 whereas the effect is observed and marked in composition Nos. 3 to 6 in Example 7 is considered to be due to the fact that the coexistence of phenylalanine and histidine with the cellulose ether derivative in composition Nos. 3 to 6 provides a marked sterilization resisting effect for a protein.

Effect of the Invention

The protein composition of the present invention has resistance to radiation sterilization. The sterile composition of the present invention retains the structure and function of a protein though it is sterilized.

Industrial Feasibility

The protein composition of the present invention is used in the manufacturing industry of medical products which requires the function and sterility of a protein.

**Claims**

1. A protein composition which comprises a mixture of glycine, phenylalanine and histidine and a cellulose ether derivative as an additive and which is sterilized with radiation.

2. The protein composition according to claim 1, wherein the additive consists of a mixture of glycine, phenylalanine and histidine and a cellulose ether derivative, and at least part of the protein is inside the cellulose ether derivative.

3. The protein composition according to claim 1 or 2, wherein the cellulose ether derivative is selected from the group consisting of hydroxypropyl cellulose, methyl cellulose, hydroxyethyl cellulose, hydroxypropylmethyl cellulose, sodium carboxymethyl cellulose and mixtures thereof.

4. The protein composition according to claim 1 or 2, wherein the cellulose ether derivative is selected from the group consisting of hydroxypropyl cellulose, hydroxyethyl cellulose, hydroxypropylmethyl cellulose and mixtures thereof.

5. The protein composition according to any one of claims 1 or 2, wherein the cellulose ether derivative is hydroxypropyl cellulose.

6. The protein composition according to any one of claims 1 to 5, wherein the protein is selected from the group consisting of enzymes, transport proteins, muscle proteins, defense proteins, toxin proteins, protein hormones, storage proteins, structural proteins, growth factors and mixtures thereof.

7. The protein composition according to any one of claims 1 to 5, wherein the protein is fibrinogen.

8. The protein composition according to any one of claims 1 to 7 which is in a film form.

9. The protein composition according to any one of claims 1 to 7 which is in a fiber form or a nonwoven fabric form.

**Patentansprüche**

1. Proteinzusammensetzung, die eine Mischung von Glycin, Phenylalanin und Histidin und einem Celluloseetherderivat als ein Additiv umfasst und die durch Strahlung sterilisiert wurde.

2. Proteinzusammensetzung nach Anspruch 1, wobei das Additiv aus einer Mischung von Glycin, Phenylalanin und Histidin sowie einem Celluloseetherderivat besteht und sich mindestens ein Teil des Proteins innerhalb des Celluloseetherderivats befindet.

3. Proteinzusammensetzung nach Anspruch 1 oder 2, wobei das Celluloseetherderivat aus der aus Hydroxypropylcellulose, Methylcellulose, Hydroxyethylcellulose, Hydroxypropylmethylcellulose, Natriumcarboxymethylcellulose und Mischungen davon bestehenden Gruppe ausgewählt ist.

4. Proteinzusammensetzung nach Anspruch 1 oder 2, wobei das Celluloseetherderivat aus der aus Hydroxypropylcellulose, Hydroxyethylcellulose, Hydroxypropylmethylcellulose und Mischungen davon bestehenden Gruppe ausgewählt ist.

**5.** Proteinzusammensetzung nach einem der Ansprüche 1 oder 2, wobei es sich bei dem Celluloseetherderivat um Hydroxypropylcellulose handelt.

**6.** Proteinzusammensetzung nach einem der Ansprüche 1 bis 5, wobei das Protein aus der aus Enzymen, Transportproteinen, Muskelproteinen, Abwehrproteinen, Toxinproteinen, Proteinhormonen, Speicherproteinen, Strukturproteinen, Wachstumsfaktoren und Mischungen davon bestehenden Gruppe ausgewählt ist.

**7.** Proteinzusammensetzung nach einem der Ansprüche 1 bis 5, wobei es sich bei dem Protein um Fibrinogen handelt.

**8.** Proteinzusammensetzung nach einem der Ansprüche 1 bis 7 in Filmform.

**9.** Proteinzusammensetzung nach einem der Ansprüche 1 bis 7 in Faserform oder in Vliesform.

## Revendications

**1.** Composition de protéines qui comprend un mélange de glycine, de phénylalanine et d'histidine et un dérivé d'éther de cellulose en tant qu'adjuvant, et qui est stérilisée par rayonnements.

**2.** Composition de protéines selon la revendication 1, où l'adjuvant est constitué d'un mélange de glycine, de phénylalanine et d'histidine et d'un dérivé d'éther de cellulose, et au moins une partie de la protéine est à l'intérieur du dérivé d'éther de cellulose.

**3.** Composition de protéines selon la revendication 1 ou 2, où le dérivé d'éther de cellulose est choisi dans le groupe constitué par hydroxypropylcellulose, méthylcellulose, hydroxyéthylcellulose, hydroxypropylméthylcellulose, carboxyméthylcellulose sodique et leurs mélanges.

**4.** Composition de protéines selon la revendication 1 ou 2, où le dérivé d'éther de cellulose est choisi dans le groupe constitué par hydroxypropylcellulose, hydroxyéthylcellulose, hydroxypropylméthylcellulose et leurs mélanges.

**5.** Composition de protéines selon l'une quelconque des revendications 1 ou 2, où le dérivé d'éther de cellulose est l'hydroxypropylcellulose.

**6.** Composition de protéines selon l'une quelconque des revendications 1 à 5, où la protéine est choisie dans le groupe constitué par enzymes, protéines de transport, protéines musculaires, protéines de défense, protéines toxiques, protéines hormonales, protéines de stockage, protéines de structures, facteurs de croissance et leurs mélanges.

**7.** Composition de protéines selon l'une quelconque des revendications 1 à 5, où la protéine est le fibrinogène.

**8.** Composition de protéines selon l'une quelconque des revendications 1 à 7, se présente sous forme d'une pellicule.

**9.** Composition de protéines selon l'une quelconque des revendications 1 à 7, se présente sous forme de fibres ou sous forme de tissu intissé.

Fig. 1

Sterilization resisting effect for protein of a combination
of a cellulose ether derivative and specific additives
(axis of ordinate: gel strength relative value (before
sterilization: 100))

Fig. 2

Evaluation results of sterilization resisting effect of a
combination of a cellulose ether derivative and specific
additives (axis of ordinate: an increase in the amount of
protein aggregate (%))

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 0437095 A
- EP 0562864 A
- US 5730933 A
- WO 2000033893 A
- JP 2011047089 A